## Europäisches Patentamt
## European Patent Office
## Office européen des brevets

(19)

(11) Publication number: **0 163 867**
**B1**

(12) **EUROPEAN PATENT SPECIFICATION**

(45) Date of publication of patent specification: **10.08.88**

(21) Application number: **85104453.7**

(22) Date of filing: **12.04.85**

(51) Int. Cl.⁴: **C 12 Q 1/04**

(54) Medium for beta-galactosidase test.

(30) Priority: **09.05.84 JP 91066/84**

(43) Date of publication of application:
**11.12.85 Bulletin 85/50**

(45) Publication of the grant of the patent:
**10.08.88 Bulletin 88/32**

(84) Designated Contracting States:
**BE DE FR GB IT**

(56) References cited:
**EP-A-0 018 825**
**GB-A-2 035 372**
**GB-A-2 099 454**

(73) Proprietor: **TERUMO KABUSHIKI KAISHA**
trading as **TERUMO CORPORATION**
**44-1, 2-chome, Hatagaya Shibuya-Ku**
**Tokyo 151 (JP)**

(72) Inventor: **Igarashi, Takeshi**
**31-13, 1-chome, Suwa**
**Tama-shi Tokyo (JP)**
Inventor: **Endo, Toshihiko**
**1417-1, Miyajima**
**Fuji-shi Shizuoka-ken (JP)**
Inventor: **Koshi, Isei**
**3350-1, Mannohara-Shinden**
**Fujinomiya-shi Shizuoka-ken (JP)**

(74) Representative: **Henkel, Feiler, Hänzel & Partner**
**Möhlstrasse 37**
**D-8000 München 80 (DE)**

Courier Press, Leamington Spa, England.

## Description

### BACKGROUND OF THE INVENTION
### Field of the Invention

This invention relates to a medium for testing microorganisms for biochemical behavior. More particularly, this invention relates to an improved medium to be used in performing $\beta$-galactosidase test on microorganisms such as enteric bacteria by the method of biochemical identification.

For administration of a proper medication to a patient of an infectious disease, it is essential to identify a pathogenic microorganism responsible for the disease, subjecting the patient to a sensitivity test, and selecting an effective medicine. In the identification of such a pathogenic microorganism, many biochemical tests are performed. One of them is a $\beta$-galactosidase test. The medium of this invention is used for the $\beta$-galactosidase test.

### Description of the Prior Art

The $\beta$-galactosidase test is intended to detect a microorganism which has an enzyme capable of being hydrolyzing $\beta$-glycosidic linkage. At present, it is one of the most important tests performed on microorganisms for biochemical behavior. This test is performed by culturing the given microorganism in a medium comprising a compound having lactose or $\beta$-glycosidic linkage. Recently, as a culture medium for use in this test, a composition comprising tryptone T, glucose, disodium monohydrogen phosphate, o-nitrophenyl-$\beta$-D-galactopyranoside, sodium hydrogen sulfite and purified water has been proposed (Japanese Patent Publication No. SHO 58(1983)—20263). This medium permits the time required for culture to be notably decreased as compared with the conventional countertype. Specifically, the culture time has been one to two days in the conventional medium, whereas it is four to five hours in the medium under discussion, making it possible to conduct the test and find the result of the test on one same day. Depending on the kind of the microorganism under test, however, the culture performed for four to five hours in this medium does not permit the reaction to proceed sufficiently for required evaluation. In this case, the culture time must be elongated and the evaluation of the test result made on the following day. In the circumstance, the desirability of developing a medium in which any microorganism can be cultured sufficiently in a matter of four to five hours to permit clear distinction between positive and negative test of the reaction has found growing approval.

For early medication, the identification of a pathogenic microorganism is desired to be carried out as rapidly as permissible. There are, however, times when the evaluation of the test result is completed to be performed on the following day because of the convenience of the work involved in the test. In this case, the medium is desired to be such that the culture time is not rigidly specified and, therefore, the culture time may be elongated to suit the convenience of the work and, despite the elongation of the culture time, the reaction is not suffered to proceed excessively and the evaluation of the test result can be conducted accurately.

For the efficiency of work involved, many biochemical tests are frequently performed all at once by the use of a multi-well culture plate. Thus, it becomes necessary for culture times of different test items to be equalized to one another. Again in this case, the culture medium is desired not to involve any rigid limitation of culture time.

An object of this invention, therefore, is to provide a novel medium for the $\beta$-galactosidase test.

Another object of this invention is to provide a medium for the $\beta$-galactosidase test which does not specifically limit culture time and enables the identification of the pathogenic microorganism to be performed on the same day as the culture is carried out or on the day following the culture.

A further object of this invention is to provide a medium for the $\beta$-galactosidase test which permits a color reaction to produce a distinct color and enables the evaluation of test result to be effected with ease.

### SUMMARY OF THE INVENTION

The objects described above are attained by a medium for the $\beta$-galactosidase test, which comprises 0.3 to 3 g of yeast extract, 1 to 10 g of peptone-tryptic hydrolysate of casein, 0.05 to 0.5 g of lactose, 0.5 to 5 g dialkali metal monohydrogen phosphate, 0.05 to 0.5 g of monoalkali metal dihydrogen phosphate and 0.5 to 5 g of o-nitrophenyl-$\beta$-D-galactopyranoside, which composition assumes a pH indicator, which composition assumes a pH value in the range of 7.2 to 7.7 when dissolved in 1 liter of water.

### DESCRIPTION OF THE PREFERRED EMBODIMENT

The medium of this invention comprises yeast extract, casein-trypsin hydrolyzing peptone, lactose, dialkali metal monohydrogen phosphate, monoalkali metal dihydrogen phosphate and o-nitrophenyl-$\beta$-D-galactopyranoside.

The composition of this medium comprises 0.3 to 3 g, preferably 0.4 to 2.5 g, of yeast extract, 1 to 10 g, preferably 2 to 9 g, of peptone-tryptic hydrolysate of casein, 0.05 to 0.5 g, preferably 0.06 to 0.4 g, of lactose, 0.5 to 5 g, preferably 0.6 to 4 g, of dialkali metal monohydrogen phosphate, 0.05 to 0.5 g, preferably 0.06 to 0.4 g, of monoalkali metal dihydrogen phosphate, and 0.5 to 5 g, preferably 0.6 to 4 g, of a o-nitrophenyl-$\beta$-D-galactopyranoside.

The medium in accordance with the present invention has a feature that dialkali metal monohydrogen

2

phosphate and monoalkali metal dihydrogen phosphate as pH adjusters control buffering ability of the medium. If the buffering ability is suppressed, it is liable to be pseudo-positive when the determination is carried out next day, because it is designed that the reaction occurs in a short time cultivation. If the buffering activity is hastened in order to avoid such defect, it becomes difficult to occur the reaction in short time cultivation. Thus it is necessary to use desired amounts of dialkali metal monohydrogen phosphate and monoalkali metal dihydrogen phosphate in order to make possible either on the same day and the following day. As the alkali metal salts thereof, potassium or sodium salts are preferable.

Color of positive of β-galactosidase reaction is yellow and that of negative is colorless, but when peptone and yeast extract is dissolved in water, it usually becomes light yellow, so it is confusing with positive. Thus it is desirable to determine the content thereof by selecting yeast extract and peptone having slight color so that the negative becomes almost colorless within the range that the reaction is not effected.

Further, when isopropyl-β-D-thiogalactopyranoside is added to the medium, the reaction is promoted. It is used in an amount of 0.01 to 0.1 g, preferably 0.03 to 0.08 g per liter of water.

The proportions of the components in the medium of this invention are critical. In particular, β-galactosidase reaction is suppressed at acidic side and occurs at alkaline side because pseudo-positive occurs, so the proportion of the dialkali metal monohydrogen phosphate and monoalkali metal dihydrogen phosphate are fixed so that the pH value of the medium will all in the range of 7.2 to 7.7, preferably 7.4 to 7.6, and the color of the color reaction will appear distinctly.

For the medium of the present invention, o-nitrophenyl-β-D-galactropyranoside proves to be the most suitable coloring substance. When it is used, the color of the color reaction is distinct enough to permit easy discrimination between positive and negative test even when the amount of the sample is small.

The medium of this invention is prepared for use by dissolving the components in their respective proportions in water. In recent years, it is normal for numerous tests to be performed all at once by the use of a multi-well culture plate. For the medium of this invention to be used in this manner, it is poured into the wells of the culture plate and dried to be used as dry medium. In the test, a suspension of a microorganism is dispensed in a prescribed volume to the individual wells and left standing therein to effect required culture for a prescribed length of time. By the change in color of the culture solution, the discrimination between positive test and negative test of the β-galactosidase test is effected.

The medium of the present invention is used in much the same way as the conventional medium for β-galactosidase test. With the medium of this invention, although the culture time can be reduced to the order of three to five hours, it is not specifically limited to that range. The culture may be performed for a longer period. Thus, the culture medium permits the evaluation of the test result either on the same day as the sample is taken or on the following day. The color produced in color test is so distinct as to permit easy discrimination between positive test and negative test of the reaction.

Now, one working example of the actual use of the medium of this invention in the β-galactosidase test and referential examples of the use of the media of this invention will be described below.

Examples I-V and Controls I-V

| composition of medium | Example | | | | | Control | | | | |
|---|---|---|---|---|---|---|---|---|---|---|
| | I | II | III | IV | V | I | II | III | IV | V |
| Yeast extract (g)* | 0.5 | 0.5 | 1.0 | 0.4 | 2.5 | — | 0.5 | 0.5 | 5.0 | 0.1 |
| Glucose (g) | — | — | — | — | — | 0.1 | — | — | — | — |
| Sodium hydrogen sulfite (g) | — | — | — | — | — | 0.2 | — | — | — | — |
| Peptone-tryptic hydrolysate of casein (g)** | 7.0 | 7.0 | 5.0 | 2.0 | 2.0 | 10.0 | 7.0 | 7.0 | 15.0 | 0.5 |
| Lactose (g) | 0.1 | 0.2 | 0.2 | 0.06 | 0.06 | — | 0.1 | 0.1 | 1.0 | 0.03 |
| Disodium monohydrogen phosphate (g) | 2.5 | 2.5 | 2.0 | 0.6 | 4.0 | 3.0 | 0.1 | 0.1 | 0.2 | 6.0 |
| Monosodium dihydrogen phosphate (g) | 0.1 | 0.1 | 0.2 | 0.06 | 0.4 | — | 0 | 2.5 | 0.02 | 1.0 |
| Isopropyl-$\beta$-D-thiogalactopyranoside (g) | 0.05 | 0.05 | 0.05 | 0.05 | 0.05 | — | 0.05 | 0.05 | 0.05 | 0.05 |
| O-nitrophenyl-$\beta$-D-galactopyranoside (g) | 1.5 | 1.0 | 2.0 | 0.6 | 4.0 | 1.5 | 1.5 | 1.5 | 0.3 | 7.0 |
| Purified water (liter) | 1 | 1 | 1 | 1 | 1 | 1 | 1 | 1 | 1 | 1 |
| pH | 7.5 | 7.5 | 7.2 | 7.2 | 7.5 | 7.5 | 7.8 | 7.1 | 7.2 | 7.7 |

---

\*   Yeast extract produced by Oxoid Corp.

\*\*   Peptone-tryptic hydrolysate of casein produced by oxoid Corp.

Culture conditions

The media of this invention and control media having the compositions as shown above were dispensed in a unit volume of 50 µl to the wells of a culture plate and dried at 40°C. Separately various microorganisms were cultured on agar culture plates at 35° to 37°C for 18 to 24 hours and suspended in 1.0 ml sterilized distilled water in a concentration of about 6 to $9 \times 10^8$ cells/ml. The suspensions were inoculated in a unit volume of 50 µl to the dry medium and, under a cover, cultured at 35° to 37°C for a prescribed length of time. By the change in color of the resultant solutions, presence or absence of the galactosidase reaction was evaluated. The results are shown in Table 1.

Table 1

| Microorganism | Media of Examples I - V | | Control medium I | | Control medium II | | Control medium III | | Control medium IV | | Control medium V | |
|---|---|---|---|---|---|---|---|---|---|---|---|---|
| | 4 to 5 hours' culture | 16 to 20 hours' culture | 4 to 5 hours' culture | 16 to 20 hours' culture | 4 to 5 hours' culture | 16 to 20 hours' culture | 4 ro 5 hours' culture | 16 to 20 hours' culture | 4 to 5 hours' culture | 16 to 20 hours' culture | 4 to 5 hours' culture | 16 to 20 hours' culture |
| Escherichia coli | + + | + + | + + | + + | + + | + + | + | + + | + | + | + | + |
| Citrobacter freundii | + + | + + | + + | + + | + + | + + | + | + | + | + | + | + |
| Morganella morganii | - | - | - | - | - | ± - - | - | - | ± - + | ± - + | ± - + | ± - + |
| Proteus mirabilis | - | - | - | - | - | ± - - | - | - | ± - + | ± - + | ± - + | ± - + |
| Enterobacter cloacae | + | + + | + | + | + + | + + | + | + + | + | + | + | + |
| Serratia liquefaciens | + + | + + | + | + | + | + + | ± - - | + + | + | + | + | + |
| Enterobacter agglomerans | + + | + + | + | + + | + + | + + | + | + + | + | + | + | + |
| Enterobacter agglomerans | ± - + | + + | ± - - | + + | + | + + | ± - - | + + | + | + | + | + |
| Shigella dysenteriae | + + | + + | + | + + | + + | + + | + | + + | + | + | + | + |

```
+       :   Color of positive test (number of +'s indicating the intensity of color)
+ - +   :   Intermediate color closer to color of positive test
± - -   :   Intermediate color closer to color of negative test
±       :   Color of negative test
```

**0 163 867**

It is noted from the results of Table 1 that the use of the media of this invention in β-galactosidase test permit accurate identification of microorganisms without reference to length of culture time. Thus, with the medium of this invention, the evaluation of test result can be carried out either on the same day as the sample is taken or on the following day, depending on the convenience of the work involved in the test.

In contrast, in the use of control media, the color produced after 4 to 5 hours' culture is not amply distinct to permit the evaluation of test result on the same day as the sample is taken.

Moreover, the fact that the medium of this invention is not rigidly restricted by the length of culture time proves highly convenient where numerous biochemical tests are carried out all at once.

## Claims

1. A medium for β-galactosidase test, comprising:
0.3 to 3 g of yeast extract,
1 to 10 g of peptone-tryptic hydrolysate of casein,
0.05 to 0.5 g of lactose,
0.5 to 5 g of dialkali metal monohydrogen phosphate,
0.05 to 0.5 g of monoalkali metal dihydrogen phosphate, and
0.5 to 5 g of o-nitrophenyl-β-D-galactopyranoside,
which culture medium exhibits a pH value in the range of 7.2 to 7.7 when dissolved in 1 liter of water.

2. A medium according to Claim 1, which comprises:
0.4 to 2.5 g of yeast extract,
2 to 9 g of peptone-tryptic hydrolysate of casein,
0.06 to 0.4 g of lactose,
0.6 to 4 g of dialkali metal monohydrogen phosphate,
0.06 to 0.4 g of monoalkali metal dihydrogen phosphate, and
0.6 to 4 g of o-nitrophenyl-β-D-galactopyranoside.

3. A medium according to Claim 2, wherein the pH value of the medium is in the range of 7.4 to 7.6.

4. A medium according to Claim 1, wherein said dialkali metal monohydrogen phosphate and monoalkali metal dihydrogen phosphate are potassium salts or sodium salts.

## Patentansprüche

1. Medium für einen β-Galactosidase-Test mit 0,3 bis 3 g Hefeextrakt, 1 bis 10 g Pepton-tryptisches Caseinhydrolysat, 0,05 bis 0,5 g Lactose, 0,5 bis 5 g Dialkalimetallmonohydrogenphosphat, 0,05 bis 0,5 g Monoalkalimetalldihydrogenphosphat und 0,5 bis 5 g o-Nitrophenyl-β-D-glactopiranosid, wobei das Kulturmedium beim Auflösen in 1 l Wasser einen pH-Wert im Bereich von 7,2 bis 7,7 aufweist.

2. Medium nach Anspruch 1, enthaltend: 0,4 bis 2,5 g Hefeextrakt, 2 bis 9 g Pepton-tryptisches Caseinhydrolysat, 0,06 bis 0,4 g Lactose, 0,6 bis 4 g Dialkalimetallmonohydrogenphosphat, 0,06 bis 0,4 g Monoalkalimetalldihydrogenphosphat und 0,6 bis 4 g o-Nitrophenyl-β-D-galactopiranosid.

3. Medium nach Anspruch 2, dadurch gekennzeichnet, daß sein pH-Wert im Bereich von 7,4 bis 7,6 liegt.

4. Medium nach Anspruch 1, dadurch gekennzeichnet, daß es sich bei dem Dialalkalimetallmonohydrogenphosphat und Monoalkalimetalldihydrogenphosphat um die Kalium- oder Natriumsalze handelt.

## Revendications

1. Milieu pour le test de la β-galactosidase, qui comprend:
de 0,3 à 3 g d'extrait de levure,
de 1 à 10 g de peptone d'hydrolyse trypsique de caséine,
de 0,05 à 0,5 g de lactose,
de 0,5 à 5 g de phosphate monoacide de métal alcalin,
de 0,05 à 0,5 g de phosphate diacide de métal alcalin, et
de 0,5 à 5 g d'o-nitrophényl-β-D-galactopyranoside,
ce milieu de culture présentant un pH d'une valeur comprise dans l'intervalle allant de 7,2 à 7,7 lorsqu'il est dissous dans un litre d'eau.

2. Milieu selon la revendication 1, qui comprend:
de 0,4 à 2,5 g d'extrait de levure,
de 2 à 9 g de peptone d'hydrolyse trypsique de caséine,
de 0,06 à 0,4 g de lactose,
de 0,6 à 4 g de phosphate monoacide de métal alcalin,
de 0,06 à 0,4 g de phosphate diacide de métal alcalin, et
de 0,6 à 4 g d'o-nitrophényl-β-D-galactopyranoside.

7

3. Milieu selon la revendication 2, dans lequel le pH du milieu possède une valeur comprise dans l'intervalle allant de 7,4 à 7,6.

4. Milieu selon la revendication 1, dans lequel ledit phosphate monoacide de métal alcalin et ledit phosphate diacide de métal alcalin sont des sels de potassium ou des sels de sodium.